# EUROPEAN PATENT SPECIFICATION

(11) **EP 0 372 730 B1**
(45) Date of publication and mention of the grant of the patent: **04.08.1993**
(21) Application number: 89311710.1
(22) Date of filing: 13.11.1989
(51) Int. Cl.: A61K 31/00, A61K 31/70

(54) **N-acetyl glucosamine as a cytoprotective agent**
N-Acetylglukosamin als zytoprotektives Mittel
N-acétylglucosamine comme agent cytoprotectif

(30) Priority: 18.11.1988 CA 583439
(43) Date of publication of application: 13.06.1990
(73) Proprietor: THE UNIVERSITY OF BRITISH COLUMBIA, Vancouver, British Columbia, V6T 1Z3 (CA)
(72) Inventor: Burton, Albert F., Vancouver British Columbia, V5W 1W3 (CA)
(74) Representative: Silverman, Warren

(56) References cited:
- EP-A- 0 178 602
- WO-A-87/02244
- DE-A- 3 602 670
- FR-A- 2 016 182
- US-A- 4 006 224
- AMERICAN JOURNAL OF GASTROENTEROLOGY, vol. 78, no. 1, pages 19-22, 1983; A.F. BURTON et al.: "Decreased incorporation of 14C-glucosamine relative to 3H-N-acetyl glucosamine in the intestinal mucosa of patients with inflammatory bowel disease"
- EUROPEAN JOURNAL OF PHARMACOLOGY, vol. 69, no. 1, pages 69-77, 1980; T. UMETSU et al.: "Effect of proglumide on glycoprotein synthesis in aspirin-induced gastric erosions in rats"
- GUT, vol. 18, no. 3, pages 219-228, 1977; M.J. GOODMAN et al.: "Glucosamine synthetase activity of the colonic mucosa in ulcerative colitis and Crohn's disease"
- HISTOCHEMICAL JOURNAL, vol. 16, no. 3, pages 235-251, 1984; P.E. REID et al.: "Chemical and histochemical studies of normal and diseased human gastrointestinal tract. I. A comparison between histologically normal colon, colonic tumours, ulcerative colitis and diverticular disease of the colon"
- HISTOCHEMICAL JOURNAL, vol. 16, no. 3, pages 253-264, 1984; P.E. REID et al.: "Chemical and histochemical studies of normal and diseased human gastrointestinal tract. II. A comparison between histologically normal small intestine and Crohn's disease of the small intestine"
- GUT, vol. 26, no. 8, pages 761-765, 1985; J.M. RHODES et al.: "Faecal mucus degrading glycosidases in ulcerative colitis and Crohn's disease"
- GUT, vol. 25, no. 5, pages 455-459, 1984; C. O'MORAIN et al.: "Organelle pathology in ulcerative and Crohn's colitis with special reference to the lysosomal alterations"
- Affidavit Dr, Dower, Affidavit Dr. Burton

## Description

This invention pertains to the novel use of N-acetyl glucosamine in the production of an orally administered medicament effective as a cytoprotective agent for treatment of degenerative diseases of mucous membrane tissues in the intestines of a human being.

Current medical mucous membrane therapy is based largely on the theory of suppressing the formation of antibodies to the body's own tissues. This includes administering corticosteroids and other pharmacological agents, all of which inhibit cell division. Their usefulness might derive from their slowing of cell proliferation, thus reducing demand for substrates present in critical supply. They are not completely effective, however, and have serious side- effects. More recently, agents affecting synthesis of prostaglandins have been introduced to increase formation of protective mucous in gastrointestinal diseases.

Two patents, as identified and discussed briefly below, have potential relevance to this subject matter.

French Patent No. 2,473,887, July 24, 1981, discloses the use of biochemical precursors of glucosamino-glycans for the treatment of vascular disorders of functional or organic origin in which there is insufficient blood flow to the limbs, for asphyxic hypoxydotic symptoms, and in cosmetology, for skin defects caused by insufficient circulation to the skin. The precursors increase the elasticity of perivascular tissue, resulting in an increase in arterio-capillary blood flow, without having a vasodilating action.

United States Patent No. 4,006,224, February 1, 1977, discloses the treatment of ulcerative colitis or regional enteritis in a mammal by administering D-glucosamine, or one of its salts. Equal or superior results to the conventional treatments of the two conditions are obtained. The dose is 20-300 mg/kg of D-glucosamine, HCl daily. In a clinical trial, a patient with Crohn's disease that was not affected by ACTH or prednisone was given D-glucosamine hydrochloride subcutaneously. The symptoms stopped after several weeks of treatment.

An article entitled "Decreased Incorporation of ¹⁴C-Glucosamine Relative to ³H-N-Acetyl Glucosamine in the Intestinal Mucosa of Patients with Inflammatory Bowel Disease", A.F. Burton and F.H. Anderson, Vol. 78, No. 1, 1983, American Journal of Gastroenterology, discloses evidence that the synthesis of glycoproteins in intestinal mucosa of patients afflicted with inflammatory bowel disease is deficient in the diseased tissues of such patient. The article discusses possible reasons for the deficiency. However. no suggestions for alleviating the deficiency are made.

According to this invention there is provided use of N-acetyl glucosamine in the preparation of an orally administered medicament for treatment of degenerative diseases of mucous membrane tissues in the intestines of a human or non-human animal, including diverticulitis, ulcerative colitis and Crohn's disease.

By use of orally administered medicaments, N-acetyl glucosamine can be fed to the human being on a daily basis. Using this method, the human being can beneficially be fed from 300 mg to 10,000 mg of N-acetyl glucosamine per day, in particular 1,000 mg to 6,000 mg of N-acetyl glucosamine per day. Specifically, the human being can be fed about 500 mg of N-acetyl glucosamine per day.

Generally, the N-acetyl glucosamine will be incorporated in a pharmaceutically acceptable carrier.

The medicament containing N-acetyl glucosamine may be fed to the human being only as required to restore the integrity of mucous membrane tissues.

A medicament which is useful for promoting integrity and normal function of mucous membrane in a human being and comprises N-acetyl glucosamine and a pharmaceutically acceptable carrier preferably contains the N-acetyl glucosamine in an amount of 500 mg to 6,000 mg per dosage unit. For some treatments, administration of as little as 500 mg N-acetyl glucosamine from single or multiple dosage units providing such quantity may be employed.

N-acetyl glucosamine, orally administered in therapeutic dosages, according to this invention, has been found to be useful in restoring the integrity of mucous membrane tissues in a human being suffering from a degenerative disease of mucous membrane tissue in the intestine and in particular enhancing the formation of protective structures against erosion in the gut due to Crohn's disease, ulcerative colitis and diverticulitis.

N-acetyl glucosamine (NAG) is an essential precursor in the body chemistry of a human being. It provides a natural, physiological means of meeting increased amino acid demand in all mucous membranes regardless of the nature of any injurious agent which may have created the demand. NAG is known to be tasteless, is easily absorbed from the gut, is utilized efficiently by the human body and does not have any known undesirable effects.

In the past, NAG has been given (for reasons not related to the subject matter of this invention) to human beings in large doses. NAG has no known ill-effects, and it is apparently utilized unchanged for synthesis of glycoproteins or, if not used, excreted in the urine (at high doses). NAG has been reported to have slight anti-tumor action and slight cholesterol-lowering effects, but otherwise it appears to be a "committed metabolite" which is utilized exclusively as a precursor for constituents of glycoproteins, glycoasaminoglycans and glycolipids.

The amino sugar, N-acetyl glucosamine (NAG) is formed in human tissues from glucosamine, which in turn is formed from glucose. It and several derivatives thereof occur in glycoproteins (GP) glycolipids (GL) and glycosaminoglycans (GAG). These macromolecules are found on the surface of cells, in the intercellular spaces and in basement membranes. They serve to bind the cells together and also act as a protective cover for some of them. The surfaces of the cells of the digestive, respiratory and genitourinary tracts typically have a coating of a glycocalyx, a coat of GP and a secreted mucous which is mostly GP. Existing evidence can be interpreted as indicating a relative deficiency of these structures in certain conditions, especially where increased cellular proliferation occurs. This is a common feature of many disorders and can increase the demand for synthesis of tissue components, for which the supply of amino sugars might be inadequate. In inflammatory bowel disease (IBD), for example, this phenomenon results in weakened mucosa which is more susceptible to erosion and ulceration.

The inventor has discovered that sufferers of Crohn's disease have a reduced level of NAG in their system. The inventor has also discovered unexpectedly that an intake of a pharmacologically acceptable amount of NAG stimulates the synthesis of protective substances, improves tissue integrity and restores tissue function in the direction of normal. Administration of NAG in therapeutic quantities has been found to be helpful in the treatment of Crohn's disease. In IBD, specifically, the conversion of glucosamine to NAG has been found to be much slower than normal. This invention, in one aspect, is directed to overcoming IBD to at least a certain extent by supplementing the sufferer's (patient's) diet with NAG.

It is known that NAG is secreted into mother's milk and stimulates the establishment of lactobacilli in the intestine of newborn infants who are breast-fed. Such flora tends to prevent establishment of less favourable bacteria and has certain advantages in the digestion of milk and in proper bowel function. A similar situation exists in vaginitis, where the prevalence of lactobacilli has a similar protective effect. However, establishment of this organism has not heretofore been successful, probably because of the lack of the "growth factor", NAG, which is required by this organism to enable synthesis of the cell wall. Consequently, providing NAG as a dietary supplement represents a physiological means of encouraging the establishment of lactobacilli in the vagina, with the attendant benefits.

Prior to conducting the following case studies, it has not been possible to predict that the administration of NAG to a person afflicted with IBD would necessarily benefit from such treatment.

### EXAMPLES

### Case History 1-JF

Over a period of two or more years, JF developed a bowel disorder that caused periods of considerable pain and discomfort. In September of 1987, JF underwent an operation for an unrelated problem and at that time the attendant physician noticed that JF had an inflamed bowel. That physician and others that JF consulted offered no particular treatment apart from a possible operation in the event that the condition, which they diagnosed as diverticulitis, became severe.

In late September, 1987, JF began taking a daily dose of N-acetyl glucosamine in an amount approximating one teaspoonful (2 grams) per day. JF dissolved this in fruit juice and sipped the juice at frequent intervals over the daytime. JF's intestinal condition improved a great deal and she had no further attacks of diverticulitis. During this time, JF under-went a series of X-rays and sigmoidoscope examinations which confirmed that JF did indeed have diverticulitis.

By the early part of December, 1987, JF was feeling so much better that she decided to reduce the amount of NAG she was consuming daily. JF did this gradually until she was down to about 1/4 of a teaspoon (0.5 grams) per day. In late December, 1987, JF had a mild recurrence of the intestinal symptoms that she had experienced previously. JF returned to a larger daily dose of NAG, about 3/4 of a teaspoon (1.5 gm) per day, and the symptoms disappeared. In late January, 1988, JF and her husband went on holidays for two weeks during which time her consumption of NAG became somewhat irregular. Shortly before returning home, JF had another recurrence of the bowel disorder - again a mild one, but the symptoms subsided about 48 hours after resuming a steady daily intake of NAG. Since then JF has maintained a daily dose of NAG amounting to about 3/4 of a teaspoon which she takes in three portions, morning, noon and at night before bed. She has been completely symptom-free since then.

When JF reduces her daily NAG dosage to less than 1/2 teaspoon a day (about 1.0 gm a day), she finds that adverse intestinal symptoms recur.

Apart from the specific symptoms of diverticulitis - bowel spasm, pain, low-grade fever, excessive flatulence, difficult bowel movements - which have all subsided, JF has been aware that a gradual change in bowel functions, which had occurred over a period of two years tending towards increased constipation, have now been reversed.

The conventional dietary recommendations for treatment and prevention of bowel disorders is a high fibre diet. JF had been following a high fibre diet for at least two years, beginning when she first noticed mild changes in bowel function but that diet did not prevent or relieve the symptoms of diverticulitis. Only the intake of about 3/4 of a teaspoon of NAG has been beneficial for eliminating these symptoms.

### Case History 2 - WWTM

WWTM was originally operated on in July, 1953 and had the ascending colon and terminal ileum removed. Since 1968 WWTM had been working in Ghana, Nigeria and Saudi Arabia and from time to time experienced gastrointestinal (GIT) upsets of varying degrees and intensity which cleared up with medication. At the time, WWTM attributed these episodes to dysentery of various forms - not at all unusual in the tropics. This may or may not have been the case. In February, 1985, WWTM experienced an episode of severe abdominal pain. The physician who attended him at the time could find no evidence of a Crohn's flare-up and attributed it to dysentery, which most people get from time to time, particularly in tropical regions. In October, 1985, on the advice of Dr. Alan Burton, WWTM started taking about 1/3 of a gram of N-acetyl glucosamine daily, dissolved in water, and have continued this ever since.

WWTM has been very happy to say that he has had literally no problems since then, with one exception, and has enjoyed excellent health in spite of residing now in what be describes as one of the worst pestholes in the world. WWTM's biochemical parameters are all normal. Serum vitamin B₁₂ was estimated recently, and that is also still normal. WWTM does not suffer from diarrhoea at all except when he eats the most potent Pakistani food, and that is short-lived. In fact, WWTM can honestly say that for the past three years, his health has been better than for many years previously. The one episode mentioned was in February, 1985 when WWTM did have a severe GIT attack which lasted for two days when he felt as though he had swallowed a pneumatic drill and could not eat or drink anything. WWTM continues to take about 1/3 gm of N-acetyl glucosamine daily, and can eat any kind of food and drink any kind of beverage. His weight is average for his height and build and remains reasonably steady.

### Case History 3 - AD

Diagnosis of Crohn's disease was made on AD in April, 1986 and surgery or steroid therapy was proposed by the attending internist, SNS. This was refused in order instead to test N-acetyl glucosamine (NAG) as a means of possibly avoiding more drastic procedures. Up to 2 grams (one teaspoon) per day were taken by AD and the symptoms appeared to be relieved after a few days. During the next few months, symptoms recurred after about one week if AD's intake of NAG was discontinued. After several months, the symptoms abated and have been in remission for over a year, with the exception of one episode in January, 1988 when there was a temporary recurrence but it responded to an increased intake of NAG. The disease remains in remission.

### Case History 4 - SB

SB has been taking N-acetyl glucosamine on advice of my physician CML for about ten years with no untoward effects. SB has been taking n-acetyl glucosamine in quantities up to 10 grams per day. On average, she takes about a gram (about 1/2 teaspoon) per day. Recent haemoglobin, white blood counts, kidney function, liver function tests, sodium, potassium and glucose levels in her body were all normal. SB's thyroid function is normal as is her urinalysis.

### Case History 5 - KC

KC has been suffering severe fatigue approaching narcolepsy with attendant confusion and memory loss. This has been attributed to a severe food allergy problem. Because of his serious food allergies, and the attendant mental afflictions, KC has been eating a synthetic diet preparation. He has also been consuming a new anti-ulcer preparation CYTOTEC,(TM) a preparation of prostaglandins which stimulate mucous secretion. He is intolerant of many foods.

KC began taking 3 grams per day of NAG in early October, 1988 and reported after two weeks that he had experienced a dramatic beneficial response. After only a few days of commencing his daily intake of NAG (3 grams), he experienced clearing of the mental symptoms and noticed an ability to tolerate many foods which he previously could not tolerate. KC has stopped taking CYTOTEC and is now taking only NAG on a daily basis. KC is completely convinced of the therapeutic benefits of a daily intake of NAG.

## Claims

1. Use of N-acetyl glucosamine in the preparation of an orally administered medicament for treatment of degenerative diseases of mucous membrane tissues in the intestines of a human or non-human animal, including diverticulitis, ulcerative colitis and Crohn's disease.

2. The use of Claim 1, wherein the N-acetyl glucosamine is incorporated in a pharmaceutically acceptable carrier.

3. The use of Claim 2, wherein the pharmaceutical formulation is in dosage unit form, each dosage unit containing from 500 to 6,000 mg of N-acetyl glucosamine.

## Patentansprüche

1. Verwendung von N-Acetylglucosamin zur Herstellung eines oral verabreichbaren Medikaments zur Behandlung degenerativer Erkrankungen des Schleimhautgewebes im Darmtrakt eines Menschen oder Tieres einschließlich Diverticulitis, ulceröser Colitis und der Crohn-Krankheit.

2. Verwendung nach Anspruch 1,
**dadurch gekennzeichnet,** daß N-Acetylglucosamin in einem pharmazeutisch akzeptablen Träger eingebracht ist.

3. Verwendung nach Anspruch 2,
**dadurch gekennzeichnet,** daß die pharmazeutische Formulierung in der Dosierungseinheitsform ist, wobei jede Dosierungseinheit von 500 bis 6000 mg N-Acetylglucosamin enthält.

## Revendications

1. Utilisation de N-acétylglucosamine pour la préparation d'un médicament administré oralement pour le traitement de la dégénérescence de la muqueuse intestinale de l'homme et d'un animal non-humain, incluant la diverticolite, la colite ulcéreuse et la maladie de Crohn.

2. L'utilisation selon la revendication 1, dans laquelle le N-acétylglucosamine est incorporé dans un support pharmaceutiquement acceptable.

3. L'utilisation selon la revendication 2, dans laquelle la formulation pharmaceutique est sous la forme d'une dose unitaire, chaque dose unitaire contenant entre 500 et 6000 mg de N-acétylglucosamine.
